(19) Europäisches Patentamt
European Patent Office
Office européen des brevets

(11) **EP 4 321 227 A1**

(12) **EUROPEAN PATENT APPLICATION**

(43) Date of publication:
**14.02.2024 Bulletin 2024/07**

(21) Application number: **22200919.3**

(22) Date of filing: **11.10.2022**

(51) International Patent Classification (IPC):
**A63B 23/18** (2006.01)    **A61B 5/08** (2006.01)
**A61B 5/00** (2006.01)

(52) Cooperative Patent Classification (CPC):
**A63B 23/185; A61B 5/0816; A61B 5/4833;
A61B 5/486;** A63B 2230/425

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **11.08.2022 PCT/CN2022/111682**

(71) Applicant: **Koninklijke Philips N.V.
5656 AG Eindhoven (NL)**

(72) Inventors:
• **JIN, Sheng
Eindhoven (NL)**
• **YIN, Bin
Eindhoven (NL)**
• **SHI, Jun
5656AG Eindhoven (NL)**
• **ZHANG, Guanqun
5656AG Eindhoven (NL)**

(74) Representative: **Philips Intellectual Property &
Standards
High Tech Campus 52
5656 AG Eindhoven (NL)**

(54) **A SYSTEM AND METHOD FOR GUIDING THE BREATHING OF A USER**

(57) A system and method is used for guiding the breathing of a user, over a plurality of time windows. A target breathing rate (pbr) is adapted over the plurality of time windows from a start target breathing rate to an end target breathing rate, by setting a target breathing rate for a next time window based on a followability measure of how well the user follows the target breathing rate in the current time window and a variability value representing how stable the breathing rate of the user is during the current time window.

FIG. 2

EP 4 321 227 A1

## Description

FIELD OF THE INVENTION

This invention relates to slow paced breathing systems and methods.

BACKGROUND OF THE INVENTION

A slow and regular breathing activity is considered beneficial for relaxation. Paced breathing is slow, deep, diaphragmatic breathing and is a technique that can be used to calm down the body and distract the mind from worries. Paced breathing is known to yield physiological changes in a person that are similar to those observed when preparing for sleep. Therefore, slow-paced breathing (SPB) has been proven to be effective at reducing stress and enhancing self-control of many body parameters, and some studies also reported that human autonomic function can be modulated by paced breathing to improve sleep.

Certain paced breathing products are available on the market using tactile, vibration or other interactive methods to guide a user to follow certain paced breathing patterns generated by the product. Usually, a fixed paced breathing pattern is integrated in the product, which means the program will start from a fixed breathing rate such as 12 bpm (breaths per minute) to another fixed rate such as 8 bpm in a certain period, or else the program keeps a fixed breathing rate.

A user may however not be able to follow the output breathing rate and hence will feel uncomfortable and even give up the training.

Adaptive breathing rate control methods have been proposed, for example which measure a user's breathing rate and adjust the output breathing rate accordingly. However, the adjustment may still not be suitable for the user, and they may need a longer time to have a stable breathing rate before they can follow a newly adjusted rate.

There is therefore a need for a more intelligent paced breathing approach.

SUMMARY OF THE INVENTION

The invention is defined by the claims.

According to examples in accordance with an aspect of the invention, there is provided a system for guiding the breathing of a user over a plurality of time windows, comprising:

a controller for controlling an actuator which provides guidance to the user to follow a target breathing rate, wherein the controller is configured to:

receive a breathing rate of the user;
set a start target breathing rate; and
adapt a target breathing rate over the plurality of time windows from the start target breathing rate to an end target breathing rate by setting a target breathing rate for a next time window based on a followability measure of how well the user follows the target breathing rate in the current time window and a variability value representing how stable the breathing rate of the user is during the current time window.

This system is used to assist a user in following a breathing pattern, in particular to perform slow paced breathing. Before the paced breathing program starts, a start breathing rate is set. It may be fixed, or the breathing rate of the user may be used to set the start target breathing rate of the generated paced breathing program.

The target breathing rate is adaptive in that the user's breathing rate is measured and a determination is made of how well the user is following the guidance, in particular based a followability measure that indicates how well the user is able to follow the target as well as a variability value indicating the stability of the user's breathing.

The adaptation is determined at the end of each time window, hence in real-time. Thus, the program is fully adaptable, since a new target is set at the end of each time window. The time window for example has a duration in the range 30 second to 2 minutes such as 1 minute. It is the time over which the values of stability are assessed, and it is the shortest time duration between successive changes in target breathing rate.

The adaptation may slow down the target breathing rate at each time window or it may keep the current target breathing rate, or even increase it. This adaptive method will improve user experience to avoid enforcing the user to follow a certain breathing rate that they cannot comfortably follow.

The end value of the target breathing rate is for example set according to best practice, for example based on research over the population. Alternatively, it can be set according to the user's health conditions and needs. The period during which the target breathing rate is adapted is for example in the range 5 to 15 minutes. The step size is then that required to reach the end target breathing rate from the start target breathing rate in the number of steps that are available. For example, the step size is (start value of target breathing rate - end value of target breathing rate)/available number of steps. In one embodiment, if the start value of the target breathing rate is lower than the end value of the target breathing rate (i.e. the user is already breathing a slow breathing pace), the system can be defined such that it will always output user start breathing rate for the run time of the system. In such a case, it is not desirable to increase the breathing rate to a higher end value of the target breathing rate.

The adaptive process can end automatically when a predefined time is expired, or when turned off manually by the user. The predefined time can be a part of the run time or can be equal to the run time of the system.

The sensed breathing rate may be the breathing rate at any particular moment in time within the current time window, for example at the end of the current time window (but it could equally be an average breathing rate over the current time window).

The controller is for example configured to set a lower target breathing rate for the next time window when the followability measure is below a followability threshold, indicating an ability to follow the target breathing rate.

In this example, a high value of the followability measure indicates poor following (e.g. large variance), but of course the followability measure may instead have a high value for good following.

The lower target breathing rate is for example obtained by reducing the target breathing rate for the current time window by a fixed amount. This provides a simple to implement process. The fixed amount may however be selected initially based on the difference between the start and end target breathing rates. The fixed amount could the step size described above.

The controller is for example configured, when the followability measure exceeds a followability threshold, indicating an inability to follow the target breathing rate, to set a target breathing rate for the next time window based on:

the variability value; and/or
a difference value representing how close the target breathing rate and a breathing rate of the user are over the current time window.

Thus, if the user cannot follow the target breathing rate fully, and the user's breathing rate still needs to be reduced, the target is adapted in an intelligent way. If the user can follow the target, the target is reduced from one time window to the next as explained above.

The difference value, representing how close the target breathing rate and the breathing rate of the user are over the time window, may for example be based on a difference between the last value of the user breathing rate in the time window and the value of the target breathing rate in the time window. In some embodiments, the difference value can also indicate which one of the target breathing rate and the breathing rate of the user is greater or lower.

The controller is for example configured to set a target breathing rate for the next time window the same as the target breathing rate in the current time window, if the difference value does not exceed a first threshold and the variability value does not exceed a second threshold.

Thus, the same target breathing rate is used even through the user cannot properly follow the target, because the variability is low and the difference is low. Thus, it is expected that the user will be able to follow the target in the next time window.

The controller may be further configured to set the target breathing rate for the next time window the same as the target breathing rate in this way only if the target breathing rate was not the same for the current time window as for the previous time window.

Thus, an extra determination may be made as to whether the current paced breathing rate will last for an extra step or not. If the user cannot follow the paced breathing rate in the current step, but the difference value is small and the variability is low (as explained above, meaning the user cannot follow the target but is close to being able to do so) the user may then also be given another step to follow. If for the next step the user still cannot follow the target but the difference value is small and the variability is low (so there have been two same targets in a row), then the target is increased to make it easier for the user to follow.

Thus, the controller may be configured in this particular situation to:

set the target breathing rate for the next time window the same as the target breathing rate in the current time window when the target breathing rate is not the same for the current time window as for the previous time window; and
set a higher target breathing rate for the next time window when the target breathing rate is the same for the current time window as for the previous time window.

The controller is for example configured to set a higher target breathing rate for the next time window if the difference value does not exceed a first threshold and the variability value exceeds a second threshold.

Thus, if the user is close to meeting the current target breathing rate but cannot achieve this with low variability, a higher target breathing rate is set.

The controller may be configured to set a higher target breathing rate for the next time window if the difference value exceeds a first threshold and the breathing rate of the user is higher than the target breathing rate in the current time window.

Thus, if the user is a long way from meeting the target breathing rate, and breathing too fast to meet the target, a higher breathing rate is set.

The higher target breathing rate is for example obtained by increasing the target breathing rate by a fixed amount. This provides a simple adaptation to the target breathing rate. The fixed amount could the step size described above.

The controller may be configured to set the breathing rate of the user in the current window as the target breathing rate for the next time window when the followability measure exceeds a followability threshold and the breathing rate of the user is below the target breathing rate in the current time window. For example, the last value of the breathing rate of the user in the current time window is selected to represent the abovementioned the breathing rate of the user in the current window. Thus, if the user cannot follow the target breathing rate but is already breathing at a lower rate than the target, the target for the next time window is set as the user's breathing rate. This speeds up the time to reach the desired stable slow breathing rate. This is a first approach for handling a user breathing rate already below the target breathing rate.

According to a second approach for handling a user breathing rate already below the target breathing rate, the controller may be configured to set the breathing rate of the user in the current time window as a target breathing rate for the next time window if the difference value exceeds a first threshold and the breathing rate of the user is lower than the target breathing rate in the current time window. For example, the last value of the breathing rate of the user in the current time window is selected to represent the abovementioned the breathing rate of the user in the current window.

In this case, the user is already meeting (and exceeding) the target by a significant amount. In this case, the target becomes the user's breathing rate at that time.

The controller is for example configured to:
set a start target breathing rate based on at least one initial breathing rate of the user in a time period before a first time window of the plurality of time windows.

The start breathing rate thus takes account of the user's initial breathing rate. Compared to a fixed gradually slowing down program (e.g. from a start breathing rate of 12 bpm to an end breathing rate 6 bpm) this provides a better personalized user experience as the user will smoothly enter the paced breathing program.

The start target breathing rate is for example set by:

  setting a mean of the at least one initial breathing rates in the time period as the start target breathing rate if a variability of the at least one initial breathing rates in the time period is below a third threshold; or setting a last initial breathing rate in the time period as the start target breathing rate if a variability of the at least one initial breathing rates in the time period is below a third threshold.

The initially sensed breathing rate may be a mean over the time period (over which there has been sufficiently low variability) or a last breathing rate for the time period.

The controller is for example configured to adapt the target breathing rate over the plurality of time windows from the start target breathing rate to the end target breathing rate within a predefined period, wherein the target breathing rate is adapted to the end target breathing rate during a first portion of the predefined period and is constant at the end target breathing rate for a rest portion of the predefined period.

By way of example, the predefined period (i.e., run time) is approximately 10 minutes (e.g. in the range 5 to 20 minutes). The adaptation of the breathing rate may take place over a period of 6 minutes (the first portion) and the final 4 minutes (the rest portion) provides guided breathing at the end target breathing rate. Alternatively, the target breathing rate is adapted during the whole predefined period.

The measure of followability may comprise any one of the following:

  a mean absolute percentage difference between the breathing rate of the user and the target breathing rate;
  a mean error;
  a root mean square error;
  a standard deviation;
  a mean absolute deviation;
  a Manhattan distance;
  a Euclidean distance;
  a correlation coefficient; or
  a cosine similarity.

One example is the mean absolute percentage difference, MAPD. This is a unit-less quantity which calculates the relative difference by scaling on the denominator. It has the advantage that the breathing rate differences at faster and slower breathing are well balanced.

The invention also provides a slow paced breathing system, comprising:

  an actuator for providing guidance to a user to follow a breathing pattern with a current target breathing rate;
  a breathing rate sensor; and
  the system as defined above.

The invention also provides a computer implemented method for guiding the breathing of a user, comprising:

  receiving a breathing rate of the user;
  setting a start target breathing rate;
  adapting a target breathing rate over the plurality of time windows from the start target breathing rate to an end target breathing rate by setting a target breathing rate for a next time window based on a followability measure of how well the user follows the target breathing rate in the current time window.

The invention also provides a computer program comprising computer program code means which is adapted, when said program is run on a computer, to implement the method defined above.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 shows a slow paced breathing system;
Fig. 2 is a flow chart to explain a first example of a method of controlling the target breathing rate in one time window;
Fig. 3 shows a fixed paced breathing program with 6 time windows;
Fig. 4 shows graphically a first example of how the control algorithm works;
Fig. 5 shows graphically a second example of how the control algorithm works; and
Fig. 6 is a modification to the flow chart of Fig. 2 to explain a second example of a method of controlling the target breathing rate in one time window.

DETAILED DESCRIPTION OF THE EMBODIMENTS

The invention will be described with reference to the Figs.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figs are merely schematic and are not drawn to scale. It should also be understood that the same reference numerals are used throughout the Figs to indicate the same or similar parts.

The invention provides a system and method used for guiding the breathing of a user, over a plurality of time windows. A target breathing rate is adapted over the plurality of time windows from a start target breathing rate to an end target breathing rate, by setting a target breathing rate for a next time window based on (at least) (i) a followability measure of how well the user follows the target breathing rate in the current time window and (ii) a variability value representing how stable the breathing rate of the user is during the current time window.

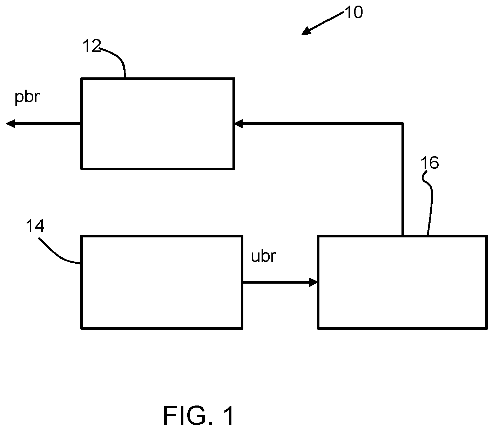

Fig. 1 shows a slow paced breathing system 10 comprising an actuator 12 for providing guidance to a user to follow a breathing pattern with a current target breathing rate pbr. A breathing rate sensor 14 provides user breathing rate information ubr. A controller 16 receives the user breathing rate information ubr and controls the actuator 12 to deliver a stimulus for guiding the user to breath at the target breathing rate pbr.

The controller 16 runs an adaptive paced breathing control algorithm which processes the user breathing rate ubr and calculates a target breathing rate pbr in real-time so that paced breathing patterns can be generated by the actuator 12 and output to the user.

To determine the user breathing rate, the system measures the breathing signal of the user. The measurement can be conducted using any sensor(s) such as based on a ballistocardiogram, or using piezoelectric pressure sensors integrated into a chest-belt, or using flow sensing. Any suitable sensor for measuring respiration may be used. The controller calculates the breathing rate of the user from the raw sensor signals of the respiration sensor.

The actuator for example may comprise an airbag. The surface of the airbag undulates (up or down) to simulate a breathing pattern and a user can touch the surface to follow the guidance. The amount of movement of the surface up and down is an amplitude of the actuator signal. Another actuator example is light that flashes slowly from dim to bright according to the target breathing rate, in which case the light intensity is an amplitude of the actuator signal. Another example is a visible waveform showing on a display presenting breathing patterns, and user can observe and follow the guidance. The amplitude may be constant for different target breathing rates, or the amplitude may also vary with the target breathing rate.

In one example, if the target breathing rate of the system is low, the amplitude is relatively high with the low frequency. If the target breathing rate of the system is high, the amplitude is relatively low with the high frequency.

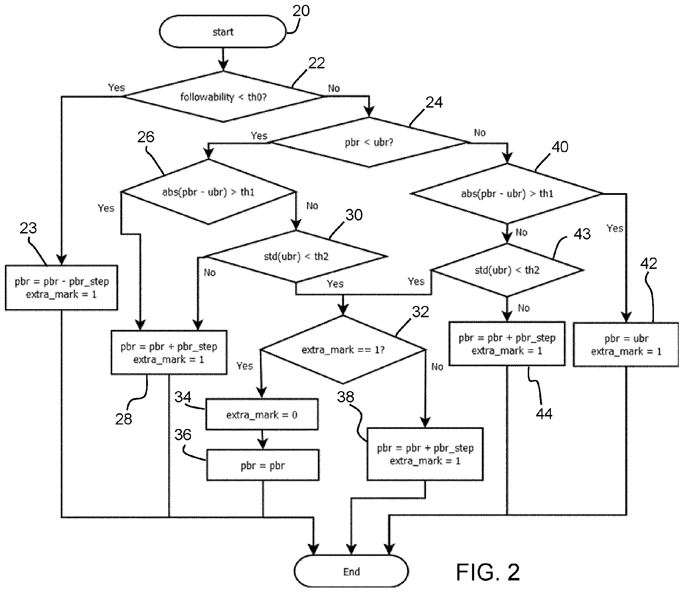

Fig. 2 is a flow chart to explain the method of controlling the target breathing rate in one time window.

The target breathing rate output to the user is controlled in a stepwise way. Thus, the system and method are for guiding the breathing of a user over a plurality of time windows, each time window forming a step in the control method. Analysis of the breathing rate during one time window is used to determine the target breathing rate for a next time window. The time window has a configurable period, for example 60 seconds. The step-based mechanism can make sure the measurement of the breathing rate, and analysis of the breathing rate as discussed below, is stable. Representative information is extracted using multiple measurements taken over the duration of the time window. The time window allows the user to have sufficient time to adapt to a new target breathing rate of the paced guided breathing.

The control mechanism logic defines how to generate the target breathing rate step by step based on certain parameters.

A first measure used to influence how the target breathing rate is adapted is a measure of measure of "followability". This measure indicates how well the user is able to follow the target breathing rate in the current time window.

The followability measure in particular quantifies the degree of the stability of the user in attempting to follow a target breathing rate. This degree of stability may be mathematically defined as a distance between the user breathing rate sequence and the paced breathing rate sequence.

In principle, any function can be employed to evaluate the followability if it can operate on two vectors (of the same length) and return a real number. Commonly used functions may include the calculation of mean absolute error, mean error, root mean square error, standard deviation, mean absolute deviation, Manhattan distance, Euclidean distance, correlation coefficient or cosine similarity.

One example is the mean absolute percentage difference (MAPD). To be more specific, MAPD can be calculated as:

$$\text{MAPD} = \frac{1}{n}\sum_{i=1}^{n}\left|\frac{\text{pbr}_i - \text{ubr}_i}{\text{ubr}_i}\right|$$

As mentioned above, pbr is the target breathing rate (i.e. paced breathing rate) in the current window set by the paced breathing method, whereas ubr is the current user breathing rate as measured. n is the number of the user breathing rates calculated within the current time window. For instance, if the time window is 60 seconds, and user breathing rate is calculated every 10 seconds, then n is 6.

MAPD is a unit-less quantity which calculates the relative difference by scaling on the denominator. It has the advantage that the breathing rate differences at faster and slower breathing will be better balanced.

While MAPD takes into account only the difference between the target breathing rate and user breathing rate over the current time window, other embodiments of the measure of followability can take into account how stable the breathing rate of the user is during the current time window and the difference to the target breathing rate over the current time window. In this example, the difference in breathing rate is summed and averaged over the time window.

The measure of followability can be updated each time a new measured breathing rate is available, or based on batch processing when a few new values have been collected. By way of example, the measure of followability can be updated at the end timestamp point of each time window.

All breathing rates within the time window are retrieved for determining the measure of followability. A user can be defined as able to follow the paced breathing if followability < th0, where th0 is a threshold with a certain value that can be predefined. By way of example, using the MAPD as defined above, th0 can be selected with a value 0.2.

Thus, this definition has a low value for a good ability to follow the target breathing rate and a high value for poor ability to follow the target breathing rate. Thus, it is a measure of deviation from the target breathing rate. Of course, the measure of followability may be defined oppositely, and comprise a measure of correlation with the target breathing rate.

The current breathing rate of the user ubr (which is compared with the target breathing rate to derive the difference abs(pbr-ubr)) is for example defined as the breathing rate as measured at the end of the current time window, although other measures may be used such as the average. The use of an average lowers the sensitivity to possible high frequency fluctuation of ubr. In another example, the current breathing rate ubr is a mean of the breathing rate only for an end segment of the current time window, for example the average over the last 10 seconds of the 60 second time window.

In addition to the overall measure of followability, there is a separate determination of a variability value (e.g. a standard deviation std(ubr)) representing how stable the breathing rate of the user is during the current time window and also determination of a difference value (abs(pbr-ubr)) representing how close the target breathing rate and the breathing rate of the user are over the current time window. Additionally, the difference value can also represent which one of the target breathing rate and the breathing rate of the user is greater or lower.

The variability value is based on a number of breathing rates of the user measured over the course of the current time window.

A first threshold th1 is used to compare with the difference value and a second threshold th2 is used to compare with the variability value. By way of example, th1 can be selected as 1.5, while th2 can be selected as 1.0.

The method starts in step 20. It is performed at the moment of the end of each time window, i.e. each step of the stepwise process. At this moment, the algorithm checks whether the followability measure is smaller than a followability threshold th0, in step 22.

If yes, hence the user is able to follow the target breathing rate in the current time window, then the paced breathing rate for the next time window will be reduced by pbr_step in step 23.

If no, hence the user is not able to follow the target breathing rate in the current time window, the adaptation of the target breathing rate depends on the target breathing rate pbr and the user breathing rate ubr, and in particular the variability value std(ubr) and difference value abs(pbr-ubr), making use of the thresholds explained above.

When the user cannot follow the current target breathing rate as determined in step 22, in step 24 it is determined if the target breathing rate is below the user breathing rate. If so, the target breathing rate has not yet been reached, and the method proceeds to step 26.

In step 26, it is determined if the difference value abs(pbr-ubr) exceeds the first threshold th1. An absolute value of the difference between the target breathing rate and the breathing rate of the user is used in this embodiment. Additionally, the target breathing rate and the breathing rate of the user are collected at the moment of the end of each time window. If the difference value abs(pbr-ubr) exceeds the first threshold th1, the user is a long way from being able to follow the target breathing rate, so the rate for the next time window is increased (to make it easier to follow) in step 28. In step 28, the breathing rate for the next window will be increased by pbr step.

The method is then complete for that time window.

If it is determined in step 26 that the difference value abs(pbr-ubr) does not exceed the first threshold th1, the user is close to the target breathing rate. It is then determined in step 30 if the variability value std(ubr) is below the second threshold th2.

If the variability value is not below the second threshold th2, it means the user's breathing rate is not stable, so the rate is also increased in step 28.

If it is determined in step 30 that the variability value is below the second threshold th2, it means the user's breathing rate is stable.

In Fig. 2, an additional variable "extra mark" is used to decide whether the current paced breathing rate will be last for an extra step or not, when the user can't follow the paced breathing rate in the current time window and the difference value abs(pbr-ubr) is smaller than the first threshold th1 (step 26 and step 40), and the variability value std(ubr) is smaller than the second threshold th2 (step 30 and step 43). This means although user cannot follow the target, they are quite close to being able to follow. The method then gives the user another time window to follow the same target breathing rate.

To implement this feature, when the system is first turned on, the value of extra_mark is 1. As is clear below, it may also be reset to 1 at some steps in the method.

In step 32, it is checked if the marker is set to 1. If it is set to 1, the marker is reset to 0 in step 34 and then the target breathing rate for the next time window is maintained at the same value as in the current time window in step 36. By resetting the parameter extra_mark to 0, if at the next time window the process again reaches step 32 (so the user still cannot follow the target breathing rate under the same scenario as in the current time window) the method will then increase the target breathing rate pbr for the next time window by pbr_step in step 38 to make the target breathing rate easier to follow. Also, in step 38, the extra_mark is reset to 1.

For all other situations, the value of the extra_mark is reset to 1 (in steps 23, 28 and 42). Thus, if the method has not reached step 32 and has not determined that the target breathing rate has remained unchanged for the current time window compared to the previous time window, whenever the method reaches step 32 in the current time window (the user cannot follow the target breathing rate but is close to being able to do so, both in terms of the difference value and the variability value), the user is given a second chance to achieve the followability measure for the target breathing rate in the current time window. Otherwise, if the method has reached step 32 and has determined the target breathing rate has already been unchanged for the current time window comparted to the previous time window, whenever the method reaches step 32 in the current time window (the user cannot follow the target breathing rate but is close to being able to do so, both in terms of the difference value and the variability value), the user will not be given a further chance to achieve the followability measure for the target breathing rate in the current time window. Instead, the target breathing rate pbr for the next time window will be increased by pbr_step and make the target easier to follow for the user.

If the user does meet the followability measure after the first repeat of pbr (in step 36), the method will follow a path to step 23 in the next step, which will reset the parameter extra_mark to value 1.

The user's breathing rate may already not be lower than the target. In step 24, if the target breathing rate is not lower than the user breathing rate, the method progresses to step 40.

The difference value is compared with the first threshold th1 in step 40. If the difference is greater than the first threshold th1, this means the user cannot follow the target breathing rate, and furthermore the breathing rate of the user is much lower than the target breathing rate. In this case, the target breathing rate for the next time window is set equal to the actual user breathing rate, in step 42. For example, the last value of the user breathing rate in the current time window is selected to represent the abovementioned actual user breathing rate in the current window.

If the user's breathing rate is too low (as determined in step 24) but by a small amount (as determined in step 40), the variability value is analyzed in step 43.

If the variability is high (not below the second threshold th2) in step 43, this means the user cannot follow the target breathing rate and has an instable breathing. In such a case, the target breathing rate is increased in step 44.

The parameter "extra_mark" is reset to 1 in steps 42 and 44.

If the variability is low (below the second threshold th2) in step 43, this means the user cannot follow the target breathing rate but has stable breathing with a breathing rate just below the target breathing rate. In such a case, the method progresses to step 32. The target breathing rate is held constant for the next time window in step 36, if the method has not previously reached step 32 and has not determined that the target breathing rate has already remained unchanged between the current time window and the previous time window (when the user cannot follow the target breathing rate but has stable breathing with a breathing rate just below the target breathing rate). Otherwise, the target breathing rate for the next time window is increased by pbr_step in step 38.

The previous description regarding steps 26, 30, 32, 34, 36 and 38 may then apply.

The main approach of the method is to help the user to control their breathing to follow from a higher breathing rate to lower breathing rate. To improve the user experience, if the user is not able to follow the target rate (according to the measure of followability), the target breathing rate is increased.

For the scenario described above, where the breathing rate of the user is lower than the target breathing rate, if the difference is smaller than the threshold, but the user is not following the breathing rate, it is possible that user breathing rate is fluctuating around target rate. The method first increases the target breathing rate to make sure the user can follow, then the method decreases the target breathing rate. If the difference between these two rates are larger than a threshold, the solution will set the target breathing rate as the user breathing rate, as this much lower user breathing rate could indicate that the user is able to breath at this rate with high probability.

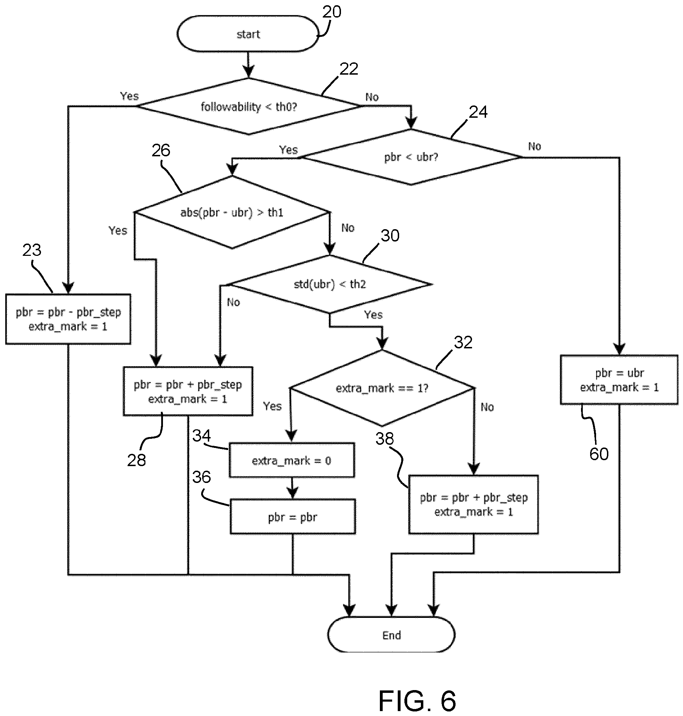

Another option is more simply to set the target breathing rate for the next time window equal to the user breathing rate if the user's breathing rate is lower than target rate and the user is not able to follow the target breathing rate in the current time window, as shown in Fig. 6.

For the first time window (after the system is turned on), the system may in one set of examples apply a standard start target breathing rate. However, more preferably, the breathing rate of the user is measured continuously to determine whether the breathing rate of the user is stable. This can be determined using the variability value over an initial time period. If the variability value (e.g. variance or standard deviation) calculated over the time period is smaller than a predefined threshold (e.g. a third threshold th3, the same or different to th2), the breathing rate may be stored as the start value of paced breathing.

The initial time period (for assessing the initial breathing rate) may be the same as the time window duration or it may be different. Multiple time periods may be analyzed until a stable value is obtained.

The start target value can be calculated as the mean value of the user's breathing rate in the stable time period or the last value of the stable time period.

The system then starts to output the paced breathing patterns with the start target breathing rate in the first step. The method then follows the method as explained above.

The upper and lower limits of the paced breathing rate can be set and checked before outputting for the next time window. For example, a lower limit value may be 5 bpm, and the logic will output 5 bpm even if the calculated new target breathing rate is lower than 5 bpm.

The system can have a user interface to allow configuring parameters for the control logic. The parameters may include all those used in the method above, or only a subset may be configurable depending on the requirement in a specific implementation.

The adaptive control of the breathing rate may be a function that can be enabled or disabled by the user. For example, the user can set a parameter "Adaptive". If set to true, then the adaptive control logic will be enabled; if set to false the algorithm will output a conventional paced breathing program from a start breathing rate to a finish breathing rate step by step.

Other parameters that may be set by the user are:

Run time: the time of the paced breathing program. The system will stop the adaptation or processing of the target breathing rate when the run time reaches this value. In one embodiment, the target breathing rate is adapted during a first portion of the run time and is constant at the end target breathing rate for a rest portion of the run time. Alternatively, the target breathing rate is adapted during the whole run time;
Time window i.e. step time: the time for each step with a certain breathing rate;
Step count: the number of steps (i.e., time windows) from the start target breathing rate to the end target breathing rate.

The personalized setting of the start target breathing rate may also be enabled or disabled. For example, the user can set a parameter "Start follow". If set to true, then before the program starts, the breathing rate of the user will be measured and set as the start target breathing rate automatically; if set to false the start breathing rate will equal to a default start value.

This default start value may also be set by the user.

The end target breathing rate may also be set by the user according to his health condition and specific needs. Alternatively, the end target breathing rate is set to an ideal comfortable breathing rate based on the research for the population.

The user may also be able to set a parameter "Finish follow". If set to true, then the end target breathing rate will be equal to the start target breathing rate times a finish percent value. If false, then the finish breathing rate will equal to a default finish value.

This finish percent value and default finish value may also be set by the user.

Step size: the amplitude adjustment for the breathing rate in each time window. Step size = (start target breathing rate - end value of target breathing rate)/step count.

Pbr_step is the increase/decrease amount for the target breathing rate in the time window in Fig. 2. It can be set as the step size.

Other settable parameters may be:

Exhale/inhale ratio: the ratio of time of exhalation phase to inhalation phase;
Amplitude start value: the amplitude of a generated breathing cycle at the start target breathing rate;
Amplitude finish value: the amplitude of a generated breathing cycle at the end target breathing rate;
Inhale hold: a hold time after inhalation;
Exhale hold: a hold time after exhalation;
Th1 value: the threshold for the difference value;
Th2 value: the threshold for the variability value.

The difference between the control approach of the invention and a standard paced breathing approach (with a fixed start target breathing rate and no measure of followability) will now be shown.

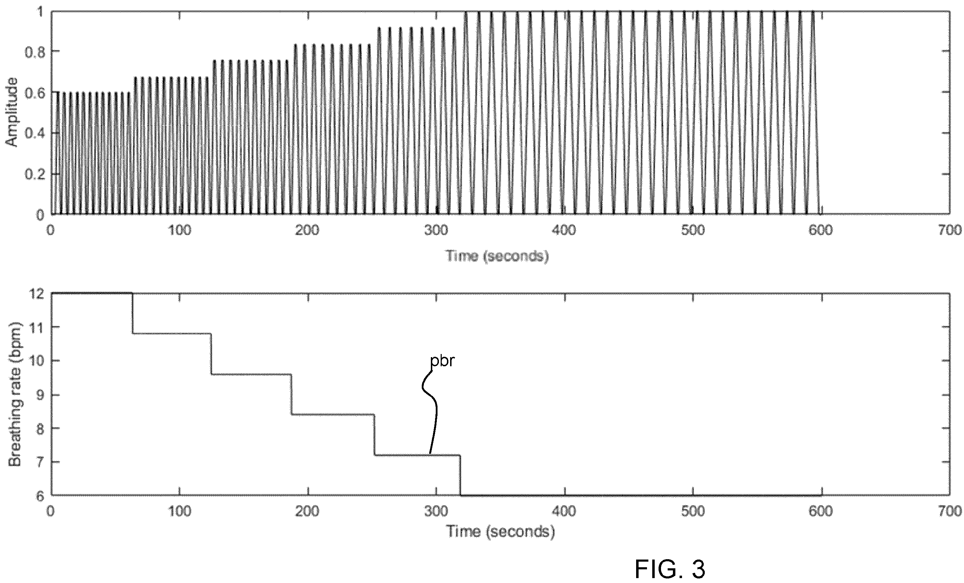

Fig. 3 shows a fixed paced breathing program with 6 time windows with a start target breathing rate of 12 bpm and an end target breathing rate of 6 bpm. The amplitude increases from 0.6 at 12 bpm to 1 at 6 bpm. 12 bpm a typical user breathing rate in the normal life. 6 bpm is a breathing rate when user feel very relaxed. In the Fig, the amplitude of 0.6 means 60% of the maximum amplitude is used, for example, while 1 means 100% of the maximum amplitude is used. The top image shows the actuator signal over time (as amplitude versus time) and the bottom plot shows the target breathing rate that is encoded by the actuator signal over time.

A user simulation model has been used to simulate the followability measure, different breathing rates.

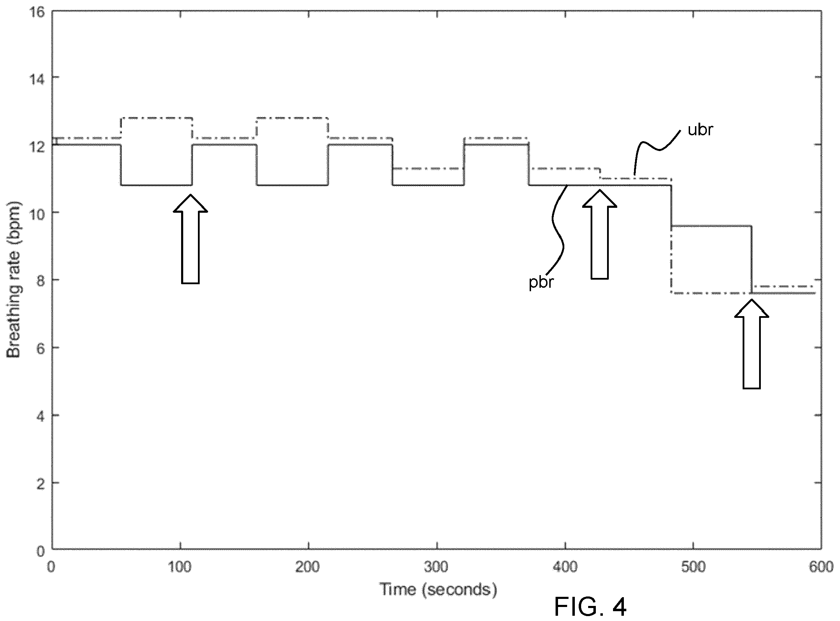

Fig. 4 shows the target breathing rate pbr (with a solid line) and the user breathing rate ubr (with a dotted line). The arrows from left to right indicate:

 (i) the target breathing rate increases (the user was not able to follow);
 (ii) an extra step with same target breathing rate is applied (the user was not able to follow but was close both in terms of difference and variability). The algorithm gives another one minute of the same target breathing rate as the previous step to let user continue to try following;
 (iii) the target breathing rate is set to the user breathing rate (because the user is breathing at a lower rate than the target). The algorithm uses the breathing rate of the user to facilitate the paced breathing, enhancing the user experience, while also accelerating the speed to reach the end breathing rate.

These verification cases show the accuracy of the control algorithm implementation, which helps the user to get more comfortable with the changes in the target breathing rate.

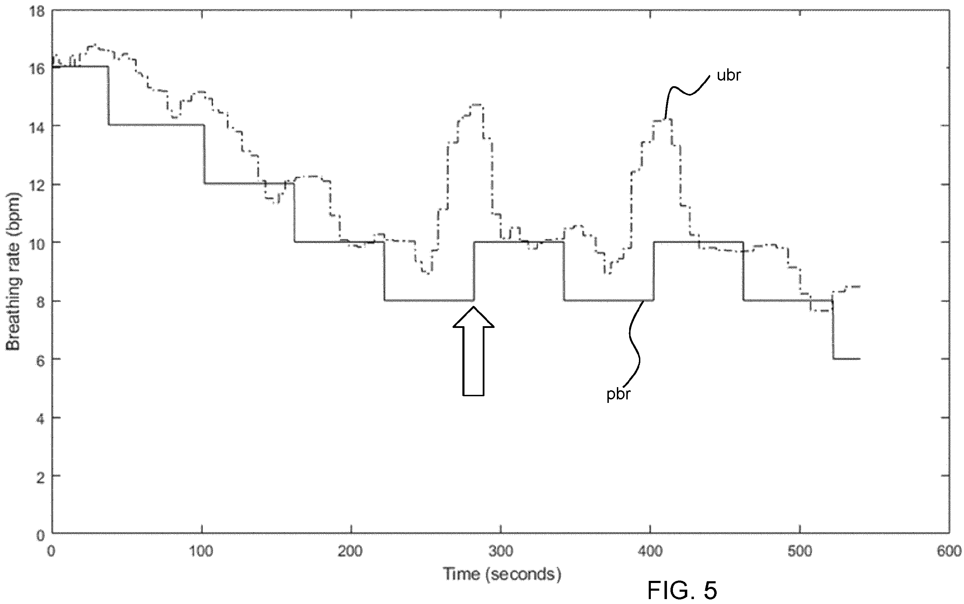

Fig. 5 shows another example of how the control algorithm works based on a real world test case showing the user breathing rate ubr and target breathing rate pbr during the paced breathing program. The target breathing rate increases or decreases according to the adaptive logic.

The user can initially follow the breathing rate and pattern generated by the control algorithm, so the output breathing rate gradually slows down. At the fifth step as shown by the arrow the user cannot follow any more so for next step the control algorithm will increase the breathing rate of the generated breathing pattern.

The time window in Figs. 3 to 5 has a duration of 60 seconds, but this is simply by way of example.

Fig. 6 shows an alternative example of the method of Fig. 2. The steps which are followed if the user breathing rate is already lower than the target breathing rate are simplified. In particular, steps 40, 42, 43 and 44 of Fig. 2 are replaced with a single step 60, whereby if the user breathing rate already equal to, or lower than, the target breathing rate, the target breathing rate is set equal to the user breathing rate. For example, the last value of the user breathing rate in the current time window is selected to represent the abovementioned user breathing rate. The other steps are identical to Fig. 2.

The invention enables a paced breathing system which outputs a guiding breathing pattern adapted to the user's breathing pattern. This improves the user experience and compliance to the paced breathing exercises and consequently improves the falling sleep efficiency.

The approach is personalized. For example, before the paced breathing program starts, the breathing rate of user is optionally measured and used to set the start target breathing rate as explained above. This enables the user to smoothly enter the paced breathing program instead of jumping to a breathing rate that could be too fast or slow for the user.

The approach is also adaptive in that the algorithm measures the user's breathing rate and calculates a followability measure to determine whether user can follow current output breathing rate generated by the paced breathing program. This adaptive method improves the user experience to avoid enforcing the user to follow a certain breathing rate that they cannot follow.

In some embodiments, breathing patterns with a same breathing rate are retained for a period, for instance 60 seconds, to give time to user to adapt to a new breathing rate and give time to obtain the user's breathing rate and calculate the followability measure.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

**Claims**

1. A system for guiding the breathing of a user over a plurality of time windows, comprising:
   a controller for controlling an actuator which provides guidance to the user to follow a target breathing rate, wherein the controller is configured to:

   receive a breathing rate (ubr) of the user;
   set a start target breathing (pbr) rate; and
   adapt a target breathing rate (pbr) over the plurality of time windows from the start target breathing rate to an end target breathing rate by setting a target breathing rate for a next time window based on a followability measure of how well the user follows the target breathing rate in the current time window and a variability value (std(ubr)) representing how stable the breathing rate of the user is during the current time window.

2. The system of claim 1, wherein the controller is configured to set a lower target breathing rate for the next time window when the followability measure is below a followability threshold (th0), indicating an ability to follow the target breathing rate.

3. The system of claim 1 or 2, wherein the controller is configured, when the followability measure exceeds a followability threshold (th0), indicating an inability to follow the target breathing rate, to set a target breathing rate for the next time window based on:

   the variability value (std(ubr)); and/or
   a difference value (abs(pbr-ubr)) representing how close the target breathing rate and a breathing rate of the user are over the current time window.

4. The system of claim 3, wherein the controller is configured to set a target breathing rate (pbr) for the next time window the same as the target breathing rate in the current time window, if the difference value (abs(ubr-pbr)) does not exceed a first threshold (th1) and the variability value does not exceed a second threshold (th2).

5. The system of claim 4, wherein if the difference value (abs(ubr-pbr)) does not exceed the first threshold (th1) and the variability value does not exceed the second threshold (th2), the controller is configured to

   set the target breathing rate for the next time window the same as the target breathing rate in the current time window when the target breathing rate is not the same for the current time window as for the previous time window; and
   set a higher target breathing rate for the next time window when the target breathing rate is the same for the current time window as for the previous time window.

6. The system of any one of claims 3 to 5, wherein the controller is configured to set a higher target breathing rate for the next time window if the difference value (abs(pbr-ubr)) does not exceed a first threshold (th1) and the variability value exceeds a second threshold (th2).

7. The system of any one of claims 1 to 6, wherein the controller is configured to set the breathing rate of the user in the current window as the target breathing rate for the next time window when the followability measure exceeds a followability threshold (th0) and the breathing rate of the user is below the target breathing rate in the current time window.

8. The system of any one of claims 3 to 6, wherein the controller is configured to set a higher target breathing rate for the next time window if the difference value (abs(pbr-ubr)) exceeds a first threshold (th1) and the breathing rate of the user is higher than the target breathing rate in the current time window.

9. The system of any one of claims 4 to 8, wherein the controller is configured to set the breathing rate of the user (ubr) in the current time window as a target breathing rate for the next time window if the difference value (abs(pbr-ubr)) exceeds a first threshold (th1) and the breathing rate of the user is lower than the target breathing rate in the current time window.

10. The system of any one of claims 1 to 9, wherein the controller is configured to:
    set the start target breathing (pbr) rate based on at least one initial breathing rate of the user in a time period before a first time window of the plurality of time windows.

11. The system of claim 10, wherein the start target breathing rate is set by:

    setting a mean of the at least one initial breathing rates in the time period as the start target breathing rate if a variability of the at least one initial breathing rates in the time period is below a third threshold; or

setting a last initial breathing rate in the time period as the start target breathing rate if a variability of the at least one initial breathing rates in the time period is below a third threshold.

12. The system of any one of claims 1 to 11, wherein the controller is configured to adapt the target breathing rate (pbr) over the plurality of time windows from the start target breathing rate to the end target breathing rate within a predefined period, wherein the target breathing rate is adapted to the end target breathing rate during a first portion of the predefined period and is constant at the end target breathing rate for a rest portion of the predefined period.

13. A slow paced breathing system, comprising:

an actuator for providing guidance to a user to follow a breathing pattern with a current target breathing rate;
a breathing rate sensor; and
the system of any one of claims 1 to 12.

14. A computer implemented method for guiding the breathing of a user, comprising:

receiving a breathing rate (ubr) of the user;
setting a start target breathing (pbr) rate; and
adapting a target breathing rate (pbr) over a plurality of time windows from the start target breathing rate to an end target breathing rate by setting a target breathing rate for a next time window based on a followability measure of how well the user follows the target breathing rate in the current time window and a variability value indicating how stable the breathing rate of the user is during the current time window.

15. A computer program comprising computer program code means which is adapted, when said program is run on a computer, to implement the method of claim 14.

FIG. 1

FIG. 2

FIG. 3

FIG. 4

FIG. 5

FIG. 6

Europäisches
Patentamt

European
Patent Office

Office européen
des brevets

# EUROPEAN SEARCH REPORT

Application Number

EP 22 20 0919

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2018/318643 A1 (KLEE MAREIKE [DE] ET AL) 8 November 2018 (2018-11-08) * the whole document * ----- | 1-15 | INV. A63B23/18 A61B5/08 A61B5/00 |
| Y | CN 104 667 487 A (ZHOU CHANG AN) 3 June 2015 (2015-06-03) * paragraphs [0067], [0073] - [0075] * ----- | 1-15 | |
| A | US 2022/080258 A1 (MANKODI HARSH A [US] ET AL) 17 March 2022 (2022-03-17) * paragraphs [0126], [0189] * ----- | 1-15 | |

|  |  |
|---|---|
|  | TECHNICAL FIELDS SEARCHED (IPC) |
|  | A63B A61B |

The present search report has been drawn up for all claims

| Place of search | Date of completion of the search | Examiner |
|---|---|---|
| The Hague | 9 August 2023 | Dhervé, Gwenaëlle |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 20 0919

This annex lists the patent family members relating to the patent documents cited in the above-mentioned European search report.
The members are as contained in the European Patent Office EDP file on
The European Patent Office is in no way liable for these particulars which are merely given for the purpose of information.

09-08-2023

| Patent document cited in search report | | Publication date | Patent family member(s) | | Publication date |
|---|---|---|---|---|---|
| US 2018318643 | A1 | 08-11-2018 | CN | 108348717 A | 31-07-2018 |
| | | | EP | 3368116 A1 | 05-09-2018 |
| | | | JP | 2018533412 A | 15-11-2018 |
| | | | US | 2018318643 A1 | 08-11-2018 |
| | | | WO | 2017072036 A1 | 04-05-2017 |
| CN 104667487 | A | 03-06-2015 | NONE | | |
| US 2022080258 | A1 | 17-03-2022 | CN | 113438924 A | 24-09-2021 |
| | | | EP | 3908183 A1 | 17-11-2021 |
| | | | US | 2022080258 A1 | 17-03-2022 |
| | | | WO | 2020146259 A1 | 16-07-2020 |

EPO FORM P0459

For more details about this annex : see Official Journal of the European Patent Office, No. 12/82